(19) Europäisches Patentamt
European Patent Office
Office européen des brevets

(11) Veröffentlichungsnummer: **0 307 777 B1**

(12) # EUROPÄISCHE PATENTSCHRIFT

(45) Veröffentlichungstag der Patentschrift: **07.01.93**

(51) Int. Cl.[5]: **C07C 205/26**, C07C 215/76

(21) Anmeldenummer: **88114593.2**

(22) Anmeldetag: **07.09.88**

Die Akte enthält technische Angaben, die nach dem Eingang der Anmeldung eingereicht wurden und die nicht in dieser Patentschrift enthalten sind.

(54) **Neue 2-Methyl-4-fluor- phenole und deren Herstellung.**

(30) Priorität: **18.09.87 DE 3731527**

(43) Veröffentlichungstag der Anmeldung:
**22.03.89 Patentblatt 89/12**

(45) Bekanntmachung des Hinweises auf die Patenterteilung:
**07.01.93 Patentblatt 93/01**

(84) Benannte Vertragsstaaten:
**CH DE FR GB IT LI**

(56) Entgegenhaltungen:
**EP-A- 0 061 741**
**DE-A- 3 524 329**
**FR-A- 1 504 228**
**US-A- 4 006 185**

(73) Patentinhaber: **BAYER AG**

**W-5090 Leverkusen 1 Bayerwerk(DE)**

(72) Erfinder: **Marhold, Albrecht, Dr.**
**Carl-Duisberg-Strasse 329**
**W-5090 Leverkusen(DE)**
Erfinder: **Fischer, Reiner, Dr.**
**Rembrandt-Strasse 15**
**W-4019 Monheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Aus der EP-A-61 741 sind aromatische Nitroverbindungen und die entsprechenden Aminoverbindungen bekannt, die zusätzlich zu der Nitro- bzw. Aminogruppe ein Fluoratom, ein Brom- oder Chloratom und eine Alkoxygruppe enthalten (s. Formel (II) auf Seite 3). Aus diesen Verbindungen lassen sich Herbizide herstellen, die deutlich weniger wirksam sind als entsprechende Herbizide hergestellt aus den Nitro- bzw. Aminogruppen enthaltenden 2-Methyl-4-fluor-phenolen der vorliegenden Erfindung.

Es wurden neue 2-Methyl-4-fluor-phenole der Formel (I) gefunden,

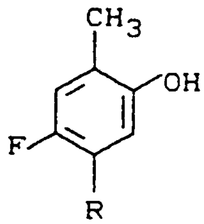

(I),

in der

R für $NO_2$ oder $NH_2$ steht.

Es handelt sich dabei um 2-Methyl-4-fluor-5-nitrophenol und um 2-Methyl-4-fluor-5-aminophenol.

Es wurde auch ein Verfahren zur Herstellung von Verbindungen der Formel (I) gefunden, das dadurch gekennzeichnet ist, daß man zunächst das phenolische Wasserstoffatom in 2-Methyl-4-fluor-phenol durch eine Schutzgruppe ersetzt, danach bei 0 bis 40 °C eine Nitrogruppe einführt, danach die Schutzgruppe durch Kochen am Rückfluß in Gegenwart von Wasser und Säure wieder abspaltet und gegebenenfalls die so eingeführte Nitrogruppe zur Aminogruppe reduziert.

Das für dieses Verfahren als Ausgangsprodukt benötigte 2-Methyl-4-fluor-phenol ist bekannt und gut zugänglich (siehe z.B. J. Am. Chem. Soc. 81, 94 (1959)).

Der erste Schritt des erfindungsgemäßen Verfahrens, der Ersatz des phenolischen Wasserstoffatoms in 2-Methyl-4-fluor-phenol durch eine Schutzgruppe kann beispielsweise so erfolgen, daß man das Wasserstoffatom durch einen Rest der Formeln (IIa) bis (IIc)

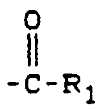

(IIa)

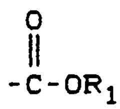

(IIb)

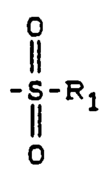

(IIc)

ersetzt, in denen

$R_1$ für $C_1$- bis $C_4$-Alkyl und bei den Formeln (IIa) und (IIc) auch für gegebenenfalls substituiertes Phenyl steht.

Solche Reste kann man beispielsweise einführen, indem man 2-Methyl-4-fluor-phenol, vorzugsweise in Gegenwart von wäßrigem Alkali und bei Temperaturen von -20 bis +100 °C mit einer Verbindung umsetzt, die einer der Formeln (IIa) bis (IIc) entspricht und an der freien Valenz ein Halogenatom, vorzugsweise ein Chloratom, enthält. Besonders bevorzugt setzt man 2-Methyl-4-fluor-phenol mit Chlorameisensäuremethyl- oder -ethylester um. Aus dem Reaktionsgemisch kann das reine Produkt z.B. isoliert werden, indem man

das Reaktionsgemisch mit einem mit Wasser nicht mischbaren Lösungsmittel, z.B. einem Chlorkohlenwasserstoff, extrahiert, den organischen Extrakt trocknet und fraktioniert destilliert.

Den zweiten Schritt des erfindungsgemäßen Verfahrens, die Einführung einer Nitrogruppe, kann man beispielsweise so durchführen, daß man das Produkt aus der ersten Stufe mit Mischsäure nitriert. Bei der Mischsäure kann es sich beispielsweise um ein Gemisch von Salpetersäure einer Konzentration von 90 bis 100 Gew.-% mit Schwefelsäure eine Konzentration von 90 bis 100 Gew.-% handeln, wobei das Gewichtsverhältnis von Salpeter- zu Schwefelsäure beispielsweise im Bereich von 1:0,5 bis 1:5 liegen kann.

Die Nitrierung wird vorzugsweise in Gegenwart eines Lösungsmittels durchgeführt, beispielsweise eines chlorierten Kohlenwasserstoffs. Bezogen auf das Produkt der ersten Stufe des erfindungsgemäßen Verfahrens kann man beispielsweise 1 bis 1,2 Mol Salpetersäure in Form der Mischsäure einsetzen.

Während der Nitrierung wird die Temperatur im Bereich von 0 bis 40 ° C gehalten. Vorzugsweise beginnt man bei Temperaturen im Bereich 0 bis 20 ° C und führt die Reaktion bei Temperaturen im Bereich 10 bis 30 ° C zu Ende. Die Aufarbeitung des Nitriergemisches kann beispielsweise so erfolgen, daß man es auf Eis gießt, die organische Phase, gegebenenfalls nach Zufügen eines in Wasser nicht mischbaren Lösungsmittels, abtrennt, trocknet und fraktioniert destilliert.

Die dritte Stufe des erfindungsgemäßen Verfahrens, die Abspaltung der am phenolischen Sauerstoffatom befindlichen Schutzgruppe, wird so durchgeführt, daß man das Produkt der zweiten Stufe in Gegenwart von Wasser und Säure am Rückfluß kocht. Hierfür geeignet sind beispielsweise wäßrige Salzsäure, wäßrige Bromwasserstoffsäure und wäßrige Schwefelsäure.

Vorzugsweise arbeitet man auch in Gegenwart eines Lösungsmittels, beispielsweise eines solchen mit einem Siedepunkt im Bereich 50 bis 120 ° C (bei Normaldruck).

Nach Beendigung der Reaktion, was im allgemeinen nach 6 bis 24 Stunden der Fall ist, kann man das Reaktionsgemisch beispielsweise wie folgt aufarbeiten:

Man engt es zunächst bei vermindertem Druck ein, fügt dann ein Extraktionsmittel, z.B. einen Ether hinzu, trennt die organische Phase ab und wäscht sie intensiv mit wäßrigem Alkali aus. Nach Ansäuern der wäßrig-alkalischen Phase fällt dann 2-Methyl-4-fluor-5-nitrophenol als Feststoff aus, den man beispielsweise aus Toluol umkristallisieren kann. Aus der verbleibenden organischen Phase kann man gegebenenfalls noch nicht verseiftes Produkt der zweiten Stufe des erfindungsgemäßen Verfahrens zurückgewinnen.

Zur Herstellung von 2-Methyl-4-fluor-5-aminophenol, falls dies gewünscht wird, ist das 2-Methyl-4-fluor-5-nitrophenol noch zu reduzieren. Diese Reduktion kann auf verschiedene Weise erfolgen, beispielsweise mit Eisen oder Zink als Reduktionsmittel. Bevorzugt wird die Reduktion katalytisch mit Wasserstoff durchgeführt. Als Katalysatoren können dabei beispielsweise Raney-Nickel oder Edelmetall-, insbesondere Palladium- oder Platin-Katalysatoren verwendet werden. Die Katalysatoren können die angegebenen Metalle in metallischer Form oder in Form von Verbindungen enthalten. Es kann sich auch um Trägerkatalysatoren handeln bei denen Metalle und/oder Verbindungen auf einem Trägermaterial, beispielsweise auf Kohle, Aluminiumoxid, Siliciumdioxid oder Silikaten aufgebracht ist. Wenn die Reduktion katalytisch mit Wasserstoff durchgeführt wird, arbeitet man vorzugsweise in Gegenwart eines Lösungsmittels, z.B. eines Alkohols wie Methanol oder Ethanol, bei Temperaturen von 20 bis 50 ° C und Wasserstoffdrucken von 1 bis 50 bar und führt die Reduktion so lange durch, bis die berechnete Menge Wasserstoff aufgenommen worden ist.

Das nach der Reduktion vorliegende Reaktionsgemisch kann man beispielsweise aufarbeiten, indem man zunächst die Folgeprodukte der Reduktionsmittel, gegebenenfalls zusammen mit überschüssigem Reduktionsmittel, oder den Katalysator abtrennt, gegebenenfalls vorhandenes Lösungsmittel abdampft und den verbleibenden Rückstand umkristallisiert, beispielsweise aus Toluol.

Es ist nicht notwendig nach jeder Stufe das dann vorliegende Produkt zu isolieren und zu reinigen. Insbesondere kann man die in der zweiten Stufe erhaltene Nitroverbindung in rohem Zustand weiterverarbeiten.

Die gute Zugänglichkeit der neuen Verbindungen 2-Methyl-4-fluor-5-nitro- und -5-aminophenol auf die zuvor beschriebene Weise war nicht zu erwarten, da bei der Nitrierung der entsprechenden Methoxyverbindung (eine $CH_3$-O- statt eine HO-Gruppe enthaltend) andere Isomere entstehen (siehe Beispiel 5).

Die neuen Verbindungen der vorliegenden Erfindung (2-Methyl-4-fluor-5-nitrophenol = A; 2-Methyl-4-fluor-5-aminophenol = B) sind wie folgt charakterisiert worden:

| | A | B |
|---|---|---|
| Schmelzpunkt | 138-142° C | 147° C |
| $^1$H-NMR | 2,55 ppm $CH_3$ sing. (3H) | 2,13 ppm Ar-$CH_3$ sing. (3H) |
| in | 6,8 ppm CH du. (1H) | 3,5 ppm $NH_2$, OH br. (3H) |
| $CDCl_3$*) | 7,5 ppm CH du. (1H) | 6,25 ppm } Ar-H du. (2H) |
| | 8,9 ppm OH sing. (1H) | 6,77 ppm |

| | A | B |
|---|---|---|
| IR | 3350 $cm^{-1}$ OH | 3380 und 3300 $cm^{-1}$ $NH_2$ |
| | 1520 $cm^{-1}$ $NO_2$ | 3500 bis 2600 $cm^{-1}$ OH (breit) |
| | | 1640 $cm^{-1}$ C=C |
| MS | 171 m/e (base peak) | |
| | 141 (24 %), 95 (38 %), 77 (42 %) | |

*) Verbindung A bei 60 $MH_z$, Verbindung B bei 100 MHz

Bei den neuen Verbindungen der vorliegenden Erfindung handelt es sich um wertvolle Zwischenprodukte zur Herstellung besonders wirksamer Herbizide und Pflanzenwachstumsregultatoren der Formel (III)

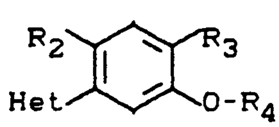

(III)

in welcher

Het für einen Heterocyclus der Formel

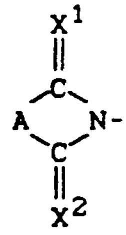 oder 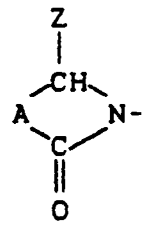

steht,

R$_2$ für Wasserstoff oder Halogen steht,

R$_3$ für Alkyl steht und

R$_4$ für jeweils gegebenenfalls substituiertes Alkyl, Alkenyl, Alkinyl oder Cycloalkyl steht oder für einen Rest

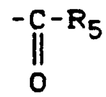

oder einen Rest -$SO_2$-R$_6$ steht,

wobei

A für einen Rest der Formel

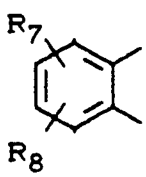 ; 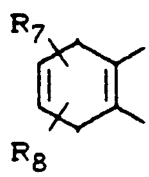 ; 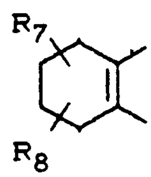 ; 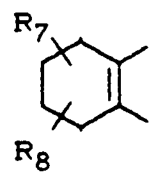 ;

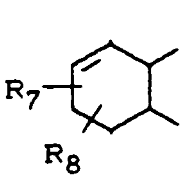 ; 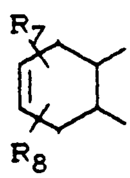 oder 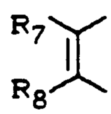

steht,

X$^1$ und X$^2$ jeweils für Sauerstoff oder Schwefel steht,

Z für Wasserstoff, Hydroxy oder Chlor steht,

R$_5$ für Alkyl, Alkoxy oder Halogenalkyl steht,

R$_6$ für Alkyl, Halogenalkyl oder für gegebenenfalls substituiertes Aryl steht und

R$_7$ und R$_8$ unabhängig voneinander jeweils für Wasserstoff, Halogen, Alkyl oder Halogenalkyl stehen.

Aus der Verbindungen der Formel (I) mit R = $NH_2$ kann man Verbindungen der Formel (III) beispielsweise erhalten, indem man die Verbindung der Formel (I) mit R = $NH_2$ mit Anhydriden der Formel (IV)

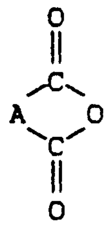

(IV),

in der

A die oben angegebene Bedeutung hat,

umsetzt und anschließend die phenolische OH-Gruppe mit üblichen Alkylierungsmitteln, Acylierungsmitteln oder Sulfonylierungsmitteln in die O-$R_4$-Gruppe überführt.

## Beispiele

### Beispiel 1

In 660 ml Wasser wurden nacheinander 42 g Natriumhydroxid und 106 g 2-Methyl-4-fluor-phenol eingetragen. Dann wurden bei +5°C 108 g Chlorameisensäuremethylester zugetropft. Es wurde für 2 Stunden bei +5°C nachgerührt, anschließend mit Dichlormethan extrahiert, die organische Phase mit Natriumsulfat getrocknet und anschließend bei vermindertem Druck fraktioniert destilliert. Es wurden 139 g (2-Methyl-4-fluor-phenyl)-methylcarbonat mit einem Siedepunkt von 98 bis 100°C bei 16 mbar und einem Brechungsindex $n_D^{20}$ von 1.4770 erhalten. Die Ausbeute betrug damit 88 % d. Th.

### Beispiel 2

65 g des Produktes aus Beispiel 1 wurden in 130 ml Dichlormethan vorgelegt und bei 20°C 65 g Mischsäure, hergestellt aus 33 Gew.-% Salpetersäure und 67 % Schwefelsäure (jeweilige Konzentration 100 %) zugetropft. Es wurde für 2 Stunden bei dieser Temperatur nachgerührt und zuletzt noch 1 Stunde bei 30°C. Nach dem Abkühlen wurde das Gemisch auf Eis gegossen, die organische Phase abgetrennt und destilliert. Es wurden 62 g (2-Methyl-4-fluor-5-nitrophenyl)-methylcarbonat mit einem Siedepunkt von 120 bis 125°C bei 0,27 mbar und einem Schmelzpunkt von 68°C erhalten. Die Ausbeute betrug 76,6 % d.Th.

### Beispiel 3

Mit 400 ml Dioxan und 300 ml wäßriger, 37 gew.-%iger Salzsäure wurden 156 g des Produktes aus Beispiel 2 12 Stunden lang auf Rückfluß erhitzt. Anschließend wurde die abgekühlte Reaktionsmischung unter vermindertem Druck eineengt und der Rückstand in Methyl-tert.-butylether aufgenommen. Die Etherphase wurde mit 400 ml 10 gew.-%iger Natronlauge intensiv gewaschen und abgetrennt. Nach dem Ansäuern der alkalischen Wasserphase fiel das Produkt aus, das abfiltriert und aus Toluol umkristallisiert wurde. Es handelte sich um 2-Methyl-4-fluor-5-nitrophenol mit einem Schmelzpunkt von 138 bis 142°C. Die Ausbeute betrug 86 g, entsprechend 73,5 % d.Th.

### Beispiel 4

54 g des Produktes aus Beispiel 3 wurden mit 800 ml Ethanol versetzt und nach Zugabe von 3 g Platindioxid bei 20 bis 30°C und einem Druck von 5 bar reduziert. Nach Aufnahme der berechneten Wasserstoffmenge wurde entspannt, der Katalysator abfiltriert, das Lösungsmittel im Vakuum abgedampft und der Rückstand aus Toluol umkristallisiert. Es wurden 36,1 g 2-Methyl-4-fluor-5-amino-phenol mit einem Schmelzpunkt von 147°C erhalten. Das entspricht 81 % d.Th.

### Beispiel 5 (nicht erfindungsgemäß)

56 g 2-Methyl-4-fluor-methoxy-benzol wurden in 150 ml Dichlormethan bei 10°C vorgelegt und 85 g Nitriersäure ($HNO_3$-Gehalt 33 %) in 30 Minuten zugetropft. Dann wurde 1 Stunde bei 10°C und 1 Stunde bei 20°C nachgerührt. Anschließend wurde das Reaktionsgemisch auf Eis gegossen, mit Dichlormethan

extrahiert, die organische Phase abgetrennt und destilliert. Das bei 130 bis 132 ° C bei 20 mbar übergehende Produkt wurde gesammelt und nach Verrühren mit 50 ml n-Hexan abgesaugt. Es wurden 26 g 2-Methyl-3-nitro-4-fluor-methoxy-benzol mit einem Schmelzpunkt von 38 bis 40 ° C erhalten.

## Patentansprüche

**1.** 2-Methyl-4-fluor-phenole der Formel

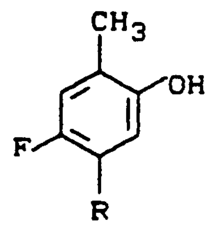

(I),

in der

R für $NO_2$ oder $NH_2$ steht.

**2.** 2-Methyl-4-fluor-5-nitro-phenol.

**3.** 2-Methyl-4-fluor-5-amino-phenol.

## Claims

**1.** 2-Methyl-4-fluoro-phenols of the formula

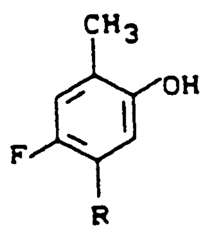

(I)

in which

R represents $NO_2$ or $NH_2$.

**2.** 2-Methyl-4-fluoro-5-nitro-phenol.

**3.** 2-Methyl-4-fluoro-5-amino-phenol.

## Revendications

**1.** 2-méthyl-4-fluorophénols de formule

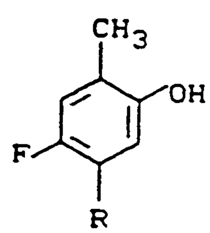

(I),

dans laquelle

R est un groupe $NO_2$ ou $NH_2$.

**2.** Le 2-méthyl-4-fluoro-5-nitrophénol.

**3.** Le 2-méthyl-4-fluoro-5-aminophénol.